Europäisches Patentamt

European Patent Office

Office européen des brevets

(11) Numéro de publication: **0 279 328 B1**

(12) **FASCICULE DE BREVET EUROPEEN**

(45) Date de publication de fascicule du brevet: **03.06.92**  (51) Int. Cl.⁵: **A61K 7/38**

(21) Numéro de dépôt: **88101861.8**

(22) Date de dépôt: **09.02.88**

(54) **Composition parfumante à action dèsodorisante ou antiperspirante.**

(30) Priorité: **20.02.87 CH 647/87**

(43) Date de publication de la demande:
**24.08.88 Bulletin 88/34**

(45) Mention de la délivrance du brevet:
**03.06.92 Bulletin 92/23**

(84) Etats contractants désignés:
**DE ES FR GB IT**

(56) Documents cités:
**CH-A- 599 787**
**FR-A- 1 600 591**
**GB-A- 1 275 969**

(73) Titulaire: **FIRMENICH SA**
**Case postale 239**
**CH-1211 Genève 8(CH)**

(72) Inventeur: **Holzner, Günter**
**15, chemin des Palettes**
**CH-1212 Grand-Lancy(CH)**

(74) Mandataire: **Salvadori, Giuseppe, Dr.**
**c/o Firmenich S.A. Case Postale 239**
**CH-1211 Genève 8(CH)**

## Description

Le parfumage de produits d'hygiène corporel, tels les désodorisants ou les antiperspirants, revêt un rôle majeur et souvent déterminant auprès du consommateur. Quoique différentes dans leur mode d'action et dans leur formulation, ces deux catégories de produits sont souvent traitées dans un même chapitre.

Si, d'une part, la fonction d'un désodorisant est de masquer l'odeur corporelle qui se développe, au niveau de l'aisselle par exemple, à l'aide d'un parfum et d'inhiber la prolifération des bactéries responsables de la formation d'odeurs désagréables typiques de la sueur, les produits vendus comme désodorisants sont souvent basés uniquement sur une solution alcoolique du parfum et d'un bactéricide. Il s'agit en général d'un milieu doux, dans lequel les composants du parfum ne risquent pas de se dégrader. Il en va autrement dans un antiperspirant dans lequel les principes actifs, en général des sels d'aluminium, développent des caractères agressifs vis-à-vis du parfum, ce qui rend ce dernier particulièrement instable. Ce phénomème entraîne des modifications de l'odeur et bien souvent également la parution de couleurs indésirables.

Le parfumeur doit s'accommoder de cet état de faits et limiter ainsi le libre choix des matières premières utilisables dans de tels systèmes.

Les produits commerciaux courants peuvent encore se distinguer en fonction de leur présentation selon qu'ils se présentent sous forme de solutions, de crème, de bâtonnet (stick) ou de poudre. Ces produits se distinguent enfin en fonction du système d'application employé. C'est ainsi qu'on distingue entre des dispositifs à aérosol, des sticks (bâtonnets), des roll-ons (dispositifs à bille) ou encore des smooth-ons. Chacun de ces dispositifs présente des caractéristiques techniques propres et le parfumage de leur principe actif requiert une solution adaptée à ces caractères distincts [voir à ce sujet par exemple: The Reheis (marque enregistrée) Report, vol.III, (1985) et Herbert P.Fiedler, Der Schweiss, Editio Cantor KG, Aulendorf i. Württ., (1968)].

Afin de pallier les inconvénients inhérents à l'agressivité du milieu vis-à-vis de l'agent parfumant, surtout dans des conditions d'acidité prononcée, l'industrie de la cosmétique s'est efforcée de développer des bases antiperspirantes actives de nature variée, basées toutefois, dans la grande majorité des cas, sur des sels d'aluminium [voir par exemple: brevet US 3,030,274; brevet NL 93662; brevet US 3,018,223; brevet FR 1,486,857]. A cet effet, il convient de mentionner le succès grandissant de bases antiperspirantes basées sur l'utilisation du Chlorhydrol (marque enregistrée de Reheis Chemical Co., Division of Armour Pharmaceutical Co.) ou du LOCRON (marque enregistrée de Hoechst AG), des chlorohydrates d'aluminium ayant un caractère modérément acide.

La pratique a montré que lors de l'emploi d'un dispositif courant, la diffusion du parfum sur l'épiderme est maximale lors de son application. Elle s'atténue progressivement au cours de la période qui s'ensuit, cette période étant plus ou moins longue en fonction de son pouvoir de rétention sur la peau de l'utilisateur. Toutefois, elle ne dépasse guère quelques heures dans le meilleurs des cas.

Afin de garantir une meilleure stabilité du parfum, l'industrie a eu recours à la technique de la microencapsulation. Dans un système microencapsulé, les ingrédients parfumants actifs sont à l'abri de l'influence exercée par les sels d'aluminium ou les métaux lourds, tels le fer ou le plomb, présents dans la base. Toutefois, de tels systèmes ne permettent pas de résoudre le problème posé par le contrôle de la diffusion du parfum. En effet, au contact avec l'épiderme et par l'action de la sueur, les microcapsules renfermant le parfum se dissolvent en libérant ses principes odorants actifs qui diffusent dans l'atmosphère environnante. Le parfum est donc percu par l'observateur de facon maximale lors d'une première transpiration, sa diffusion s'atténuant avec le temps par la suite. En se dissolvant, les microcapsules cessent en outre d'exercer leur action protectrice et le parfum se trouve donc exposé à l'action de la base antiperspirante acide.

Le brevet GB 1,275,969, publié le 1er juin 1972, décrit une composition désodorisante sous forme d'aérosol constituée par des capsules désodorisantes dans un mélange propulseur. Les capsules sont caractérisées par le fait d'avoir des parois solubles dans l'eau mais insolubles dans le mélange propulseur et imperméables à celui-ci. Une telle composition possède la propriété de maintenir, tout au moins en partie, les composants désodorisants actifs dans un milieu stable jusqu'à ce que ces mêmes ingrédients soient libérés sous l'action de la transpiration. Il s'agit en quelque sorte d'un mélange répondant à l'activation exercée par l'utilisateur même. A l'expérience, il s'est avéré qu'un tel système pouvait offrir une utilisation satisfaisante mais limitée toutefois à une seule activation. Une fois dissoutes, les capsules pouvaient libérer les ingrédients désodorisants actifs et leur action pouvait se prolonger jusqu'à leur complète évaporation.

De même, le brevet FR-A-1 600 591 décrit des particules sèches obtenues par atomisation d'une composition contenant un hydrolysat d'amidon en tant qu'agent d'encapsulation d'un liquide, notamment d'une huile aromatisante volatile. Cette composition peut éventuellement contenir un agent émulsifiant. L'hydrolysat d'amidon doit avoir un équivalent de dextrose inférieur à 40 pour permettre de former des parois plus épaisses autour de l'huile encapsulée que celles que l'on pouvait obtenir au préalable avec la gomme arabique. Les capsules ainsi obtenues lorsque mises en contact avec l'eau se dissolvent immédiatement et libèrent d'un coup les huiles encapsulées.

On connaît également du brevet CH 599 787 une composition où le parfum est toujours à l'état libre et c'est la base antiperspirante qui se trouve encapsulée dans un produit de dégradation de l'amidon ayant un contenu prépondérant en amylopectine. De la même façon que dans le cas des références citées précédemment, l'encapsulation du principe actif, dans ce cas l'antiperspirant, permettait de prolonger son effet par rapport à ce que l'on observait lorsqu'il n'était pas encapsulé, mais, une fois les capsules dissoutes dans l'eau, ce principe actif se libérait d'un seul coup.

Nous avons constaté que, par un choix particulier des ingrédients entrant dans la composition désodorisante, il était possible de provoquer un phénomène réversible de "réencapsulation" des ingrédients désodorisants actifs, de telle sorte que plusieurs activations successives pouvaient avoir lieu sur l'épiderme même sans que l'utilisateur ait eu à procéder à de nouvelles applications. Il y a donc véritablement une réencapsulation in situ lors de la phase de séchage de l'épiderme qui suit la période de transpiration.

La présente invention a ainsi pour objet une composition parfumante à action désodorisante ou antiperspirante, pour utilisation au moyen d'un dispositif d'application sur la peau, ladite composition contenant une base désodorisante ou antiperspirante active et une base parfumante, cette dernière étant combinée avec un support solide filmogène et un agent émulsifiant pour former une émulsion aqueuse laquelle, lorsque séchée sur la peau, engendre l'encapsulation in situ de la base parfumante, de façon à empêcher la diffusion de celle-ci jusqu'à ce qu'un premier accès de transpiration se produise, suivie de la réencapsulation in situ de ladite base parfumante lors du séchage subséquent de la peau.

Une telle composition présente l'avantage de permettre le contrôle de l'activation et de la diffusion du parfum dans le temps et concilie la nécessité de protéger les principes actifs du parfum et le souhait d'en prolonger la période de diffusion. Cette double action résulte du phénomène expliqué plus haut. Lors de l'application sur l'épiderme, la composition est d'abord retenue à la surface par adhésion de l'émulsion, et ce grâce à l'effet de liaison exercé par la substance de support. Sous l'effet du séchage qui s'ensuit, et qui s'effectue par simple exposition à l'air de la peau ainsi traitée, effet favorisé par la chaleur du corps, la base parfumante active est retenue sous forme de gouttelettes microscopiques enrobées d'une couche protectrice hydrosoluble constituée par le support désormais sec.

Il s'agit en l'occurrence d'un système simple qui ne nécessite pas la mise en application de dispositifs spéciaux pour son emploi. Tout système conventionnel d'utilisation courante en cosmétique, et utilisé couramment pour l'application de désodorisants et antiperspirants, peut être employé. On peut mentionner à cet effet les crèmes, les roll-ons, les smooth-ons ou encore les poudres.

Il en va de même lorsque la base parfumante active est employée sous forme microencapsulée, généralement en suspension dans un solvant alcoolique. La base parfumante, combinée préalablement à un support solide filmogène et à un agent émulsifiant, est atomisée selon les techniques usuelles dans une tour de type "spray-drier". Les microcapsules obtenues renfermant la base parfumante sont mélangées à une base désodorisante ou antiperspirante et ensuite suspendues dans une base constituée essentiellement par des cires, selon la technique employée pour la fabrication des sticks, ou dans un mélange propulseur pour la fabrication d'aérosols.

A titre de support solide filmogène, on peut utiliser l'acétate de polyvinyle, l'alcool polyvinylique, les dextrines (naturelles ou modifiées), l'amidon (naturel ou modifié), les gommes végétales, les alginates, les carraghénanes, les pectines, les xanthanes, ou encore les dérivés de la cellulose telles par exemple la carboxyméthylcellulose, la méthylcellulose et l'hydroxyéthylcellulose. Il s'agit de composés qui peuvent être définis sous le terme générique de "gommes" (voir à cet effet la définition donnée dans Kirk-Othmer, Encyclopedia of Chemical Technology, 2ème édition, vol. 10, p. 741).

Nous trouvons donc sous ce terme des gommes naturelles, tels par exemple la gomme arabique, des extraits d'algues, par exemple l'agar, les carraghénanes, le furcellarane et des gommes modifiées ou semi-synthétiques. Il s'agit de dérivés de la cellulose et de l'amidon ainsi que de gommes de fermentation microbienne, tels les lipohétéropolysaccharides, par exemple les biopolymères connus sous le nom d'émulsans (voir demande de brevet européen n° 178'443, publiée le 23.04.86).

A titre d'agent émulsifiant, on peut utiliser des mono- ou diglycérides d'acides gras, les esters dérivés de la combinaison d'acides gras avec le sorbitol ou un monosaccharide, ou leurs dérivés alcoxylés, ou un ester de l'acide tartrique, citrique, ascorbique ou lactique.

La composition suivant l'invention contient également une base parfumante. Aux termes de la présente invention, on entend par "base parfumante" toute substance ou mélange de substances parfumantes, tant à

3

l'état isolé qu'en solution ou suspension, dans leurs diluants, dissolvants ou coingrédients habituels. Ce terme comprend en particulier des solutions organiques généralement non-miscibles à l'eau dotées d'une tension de vapeur appréciable. De telles bases parfumantes peuvent être constituées par des composés appartenant à des classes chimiques distinctes et comprennent par exemple des esters, des éthers, des alcools, des aldéhydes, des cétones, des acétals, des nitriles, des hydrocarbures terpéniques, des composés hétérocycliques azotés ou soufrés ainsi que des huiles essentielles d'origine naturelle. Le choix particulier de la base parfumante dépend de l'effet odorant recherché, de la nature du produit que l'on désire parfumer et bien entendu du goût et préférence du parfumeur créateur.

Des exemples typiques de composés parfumants utiles sont donnés dans la littérature et, à cet effet, on peut citer S. Arctander, Perfume and Flavor Chemicals, Montclair, N.J. (USA), 1969.

La composition selon l'invention peut également contenir des agents bactéricides à action désinfectante ou germicide, ainsi que des agents bactériostatiques.

Quoiqu'il soit difficile de définir une gamme précise de concentration de ses constituants, la composition parfumante de l'invention contient à titre préférentiel (en poids)

2 à 20% de support solide filmogène,

0,1 à 10% d'agent émulsifiant,

0,1 à 5% de base parfumante,

5 à 25% de base désodorisante ou antiperspirante active,

le restant étant représenté par de l'eau, des dissolvants inertes et/ou des excipients, le cas échéant des agents désinfectants, germicides ou bactériostatiques.

Par base désodorisante, on entend une substance capable de masquer l'odeur corporelle et d'inhiber la prolifération des bactéries responsables de la dégradation de la sueur. Bon nombre de produits bactéricides et bactériostatiques sont connus et utilisés à cet effet. A titre d'exemple, on peut mentionner l'hexachlorophène, le dichlorophénol, le trichlorosalicylanilide (Anobial), le tribromosalicylanilide (TBS), le tétrachlorosalicylanilide (TCSA) et le trichlorocarbanilide (TCC).

A titre de base antiperspirante, on peut utiliser de préférence des sels d'aluminium, par exemple le chlorhydrate d'aluminium mentionné plus haut. Différentes compositions sont proposées sur le marché en tant que produit de base antiperspirante; le Chlorhydrol, le Chloracel et le Rezal (marques enregistrées de Reheis Chem. Co., USA) en sont des exemples. Il s'agit de sels complexes d'aluminium ou aluminium et zirconium. D'autres bases antiperspirantes sont décrites dans la littérature spécialisée (voir par exemple: Herbert P. Fiedler, Der Schweiss, Editio Kantor KG, Aulendorf i.Württ., RFA).

La composition parfumante de l'invention convient tout particulièrement à la manufacture d'articles destinés aux soins corporels. Ces derniers peuvent se présenter sous une multitude de formes différentes. On a eu l'occasion de mentionner plus haut les bâtonnets (sticks), les roll-ons (dispositifs à bille), les smooth ons, ou encore les aérosols ou les vaporisateurs à pression mécanique ou manuelle.

La composition selon l'invention est obtenue par mélange de ses différents ingrédients au moyen d'appareillage conventionnel. La technique de mélange est en soi connue et toute explication détaillée est ici superflue. On précisera que la méthode dépend essentiellement de l'article final que l'on désire faconner. C'est ainsi que par exemple, si l'on désire préparer une composition antiperspirante destinée à être employée à l'aide d'un dispositif à bille du type roll-on, l'on peut procéder de la sorte.

On verse à température ambiante la poudre constituée par le support solide filmogène, par exemple un mélange de maltodextrines, dans la quantité requise d'eau déminéralisée. Après dissolution complète, on ajoute à la solution obtenue la base antiperspirante et le tout est brassé et chauffé à 70°C puis, à cette même température, on ajoute l'agent émulsifiant en se servant d'un mélangeur homogénéisateur. Après quelques minutes de brassage, on refroidit le mélange à température ambiante et on y ajoute la base parfumante à environ 40°C. La masse visqueuse parfumée est enfin versée dans des récipients à bille du type roll-on.

Si l'on désire préparer une composition antiperspirante humide destinée à être employée à l'aide d'un dispositif de vaporisation à pression mécanique ou manuelle, on procèdera de préférence ainsi.

On verse à température ambiante le support solide en poudre dans l'eau et brasse pendant environ 1 heure jusqu'à dissolution complète. A la solution résultante, on ajoute sous agitation la base antiperspirante active (par exemple de l'hydroxychlorure d'aluminium) dans l'éthanol, puis la base parfumante préalablement mélangée à un émulsifiant. Le mélange est ensuite versé dans des récipients vaporisateurs.

L'invention est illustrée par les exemples suivants dans lesquels les températures sont indiquées en degrés centigrades et les abréviations ont le sens usuel dans l'art.

Exemple 1

Composition antiperspirante pour roll-on

Une composition antiperspirante de type crémeux destinée à être incorporée dans un distributeur à bille de type roll-on a été préparée en ajoutant par petites portions un mélange constitué par

8,90 g de Glucidex 21 (maltodextrine DE 20-23)[1)]
1,00 g de Nadex 722 (maltodextrine DE 9-12)[2)]
0,10 g d'alginate de sodium
à 65g d'eau déminéralisée.

Le mélange est brassé pendant 2 heures jusqu'à dissolution complète des ingrédients, puis 20 g de Locron (Locron L, hydroxychlorure d'aluminium, solution à 50% Hoechst AG) y ont été ajoutés. Après avoir porté la température du mélange à 70°, on a ajouté 4 g d'Emulgade 1000 NI (cire autoémulsionnante, nonionogène, Henkel AG) sous vigoureuse agitation à l'aide d'un homogénéisateur Ultra Turrax. Après quelques minutes d'homogénéisation, on a refroidi à température ambiante et ajouté 1 g de parfum (Vera 72276/B, type eau-de-Cologne, Firmenich SA, Genève) à environ 40°. Le mélange obtenu a été enfin versé dans des emballages distributeurs de type roll-on.

Exemple 2

Composition antiperspirante pour roll-on

Une composition antiperspirante de type crémeux destinée à être incorporée dans un distributeur à bille de type roll-on a été préparée en ajoutant par petites portions 10 g de Capsul (amidon de maïs modifié, National Starch) à 63,95 g d'eau déminéralisée. Après 2 h de brassage, on a ajouté un mélange constitué par:

0,10 g de Tween 20 (sorbitanmonolaurate éthoxylé ICI, Atlas)
1,00 g de Triton CG 110 (alkylglucoside, Röhm & Haas)
et 0,05 g d'Emulsan (Petroferm, biopolymère),
puis 10 g de Locron L (Hoechst AG) y ont été ajoutés et le tout a été chauffé à 70°C. A cette température, on a additionné 4 g d'Emulgade 1000 NI (Henkel AG) sous vigoureuse agitation à l'aide d'un appareil homogénéisateur. Après quelques minutes de brassage, on a refroidi à température ambiante et 1 g de parfum (Surf 635.040 E, de type aldéhydé, fleuri, vert, Firmenich SA, Genève) y a été ajouté. Le mélange obtenu a été enfin versé dans des récipients distributeurs de type roll on.

Exemple 3

Compositions antiperspirantes pour roll-on

Des compositions antiperspirantes d'aspect limpide destinées à être incorporées dans des distributeurs à bille de type roll-on ont été préparées comme indiqué aux exemples précédents en utilisant les ingrédients suivants (parties en poids):

1) Roquette    Frères

2) Grain   Processing     Corp.

5

|  |  | a | b | c |
|---|---|---|---|---|
| I. | Eau déminéralisée | 39,0 | 51,6 | 56,6 |
| II. | Natrosol 250 H [1] | 0,5 | 0,4 | 0,4 |
|  | Glucidex 21 [2] | 10,0 | 7,2 | 4,0 |
|  | Glucidex 6 [3] | - | - | 4,0 |
|  | Nadex [4] | - | 0,8 | - |
| III. | Locron L [5] | 20,0 | 20,0 | 20,0 |
|  | 1,3-Butylène-glycol | 1,5 | 1,0 | 1,0 |
|  | Ethanol 95° | 26,0 | 16,0 | 10,0 |
|  |  | a | b | c |

(suite)

|  |  | a | b | c |
|---|---|---|---|---|
| IV. | Parfum [6] | 1,0 | 1,0 | 1,0 |
|  | Cremophor RH 40 [7] | 2,0 | 2,0 | - |
|  | Lamacit 877 [8] | - | - | 3,0 |
|  |  | 100,0 | 100,0 | 100,0 |

[1] hydroxyéthylcellulose, Hercules Co.

[2] voir exemple 1

[3] maltodextrine DE 5-8, Roquette Frères

[4] voir exemple 1

[5] voir exemple 1

[6] Gabriela 230.183 de type vert, floral, fruité, Firmenich SA, Genève

[7] huile de ricin hydrogéné et éthoxylé, BASF AG

[8] nonylphénol éthoxylé, Chem. Werke Grünau

On a versé la partie II dans l'eau et on a brassé pendant 1h jusqu'à ce que le mélange soit parfaitement limpide. Les parties III et IV ont été ensuite ajoutées successivement à la solution obtenue sous bonne agitation et le tout a été versé dans des récipients distributeurs roll-ons.

Exemple 4

Composition antiperspirante pour smooth-on

Une composition antiperspirante destinée à être incorporée dans un récipient de type smooth-on a été préparée à l'aide des ingrédients suivants:

| | | |
|---|---|---|
| I. | Eau déminéralisée | 37,0 |
| II. | Glucidex 21 [1] | 9,0 |
| | Nadex [2] | 1,0 |
| III. | Locron L [3] | 40,0 |
| IV. | Emulgade 1000 NI [4] | 8,0 |
| | Arlacel 165 [5] | 4,0 |
| V. | Parfum [6] | 1,0 |
| | | ——— |
| | | 100,0 |

[1] voir exemple 1

[2] - idem -

[3] - idem -

[4] - idem -

[5] glycérylstéarate + PEG 1000 stéarate, ICI Atlas

[6] Ambrosia UN 110.381/B, de type fleuri, musqué, boisé, Firmenich SA, Genève

La partie II a été dissoute dans l'eau déminéralisée et à la solution obtenue on a ajouté la partie III, puis l'on a chauffé à 70°. Au mélange résultant, on a ensuite ajouté la partie IV préalablement chauffée à 70°. Après brassage énergique dans un appareil homogénéisateur, on a refroidi le tout et le parfum y a été ajouté à 40° environ sous agitation.

Exemple 5

Composition antiperspirante pour spray à pression

Une composition antiperspirante destinée à être employée au moyen d'un appareil distributeur comportant un système gicleur mécanique ou à pression manuelle ("pumpspray" ou "squeeze bottle") a été préparée à l'aide des ingrédients suivants:

| | | |
|---|---|---:|
| I. | Eau déminéralisée | 39,0 |
| II. | Glucidex 21 [1] | 10,0 |
| III. | Locron L [2] | 20,0 |
| | 1,3-Butylène-glycol | 1,5 |
| | Ethanol 95° | 26,5 |
| IV. | Parfum [3] | 1,0 |
| | Cremophor RH 40 [4] | 2,0 |
| | | ‾‾‾‾‾ |
| | | 100,0 |

[1] [2] voir exemple 1

[3] Diabolo UN 110.382/B, de type fleuri, vert, hespéridé, Firmenich SA, Genève

[4] voir exemple 3

La partie II a été versée dans l'eau déminéralisée et brassée jusqu'à l'obtention d'une solution claire(1 h). III et IV ont été ensuite ajoutées successivement sous agitation et le mélange résultant a été versé dans des récipients gicleurs.

Exemple 6

Composition antiperspirante pour spray aérosol

Une composition antiperspirante destinée à être appliquée au moyen d'un distributeur de type "spray" a été préparée par atomisation du mélange suivant:

| | **a** |
|---|---:|
| Eau | 49,0 |
| Glucidex 21 [1] | 36,0 |
| Nadex [2] | 4,0 |
| Alginate de sodium | 0,8 |
| Capsul [3] | – |
| Tween 20 | 0,2 |
| Parfum [4] | 10,0 |
| | ‾‾‾‾‾ |
| | 100,0 |

[1] [2] voir exemple 1

[3] amidon de maïs modifié, National Starch

[4] Surf 635.040 E, Firmenich SA, Genève

Pour effectuer l'atomisation, on a recours à un appareil de type Leaflash (CCM Sulzer):

| débit de l'émulsion | 50 kg/h |
|---|---|
| air de séchage | 320 m$^3$/h à 350°C et 0,45 bar |

La composition de base obtenue a ensuite servi à la préparation de compositions antiperspirantes pour sprays aérosol par mélange avec les ingrédients suivants:

| | | |
|---|---|---|
| **I.** | **Composition de base (a)** | **3,00** |
| | **Hydroxychlorure d'aluminium en** | |
| | **poudre micronisé (Hoechst)** | **4,20** |
| | **Allantoinate d'hydroxychlorure** | |
| | **d'aluminium (Merck)** | **0,50** |
| | **Myristate d'isopropyle** | **6,85** |
| | **Aerosil 200 (Degussa)** | **0,25** |
| | **Irgasan DP 300 (Ciba-Geigy)** | **0,20** |
| | **Propulseur 11** [1] | **50,00** |
| **II.** | **Propulseur 12** [2] | **5,00** |
| | **Propane/Butane** [3] | **30,00** |
| | | **————** |
| | | **100,00** |

**1) monofluorotrichlorométhane**

**2) difluorodichlorométhane**

**3) mélange 3,7 bar**

On a mélangé préalablement la partie I afin d'obtenir une suspension homogène et on a ensuite versé le tout dans un récipient aérosol avec adjonction de la partie II.

Exemple 7

Composition antiperspirante pour smooth-on

Une composition antiperspirante pour smooth-on a été préparée à l'aide des ingrédients suivants (parties en poids) :

| I. | Alcool cétylique | 9,0 |
|---|---|---|
| | Cire d'abeilles | 4,5 |
| | Acide stéarique | 4,5 |
| | Finsolv TN [1] | 10,0 |
| | Arlacel 165 [2] | 5,4 |
| II. | Hydroxychlorure d'aluminium en poudre micronisée [3] | 20,0 |
| | Talc | 5,0 |
| III. | Dow Corning fluid 345 [4] | 35,6 |
| IV. | Composition de base (a) [5] | 6,0 |
| | | 100,0 |

1) benzoate d'alcool C12-C15, Finetex
2) voir exemple 4
3) voir exemple 6
4) huile de silicone volatile
5) voir exemple 6

On a chauffé la partie I à 80° jusqu'à fusion complète de tous ses ingrédients et au mélange fondu on a ajouté successivement les parties II, III et IV sous bonne agitation. On a laissé ensuite refroidir à 40-50° et on a versé dans des récipients de type smooth-on.

Exemple 8

Composition antiperspirante pour bâtonnets

Une composition antiperspirante pour bâtonnets secs ("dry sticks") a été préparée à l'aide des ingrédients suivants (parties en poids) :

| I. | Octadécanol | 19,0 |
|---|---|---|
| | Arlacel 165 [1] | 1,0 |
| | PEG 1000 [2] | 5,0 |
| II. | Aerosil 200 [3] | 1,4 |
| | Talc | 1,0 |
| | Rezal 36 P [4] | 19,0 |
| III. | Dow Corning Fluid 345 5) | 47,6 |
| IV. | Composition de base (a) [6] | 6,0 |
| | | 100,0 |

1) voir exemple 4
2) polyglycol 1000, Hoechst
3) Degussa
4) complex zirconium chlorhydrate, Reheis Chem. Co.
5) huile de silicone volatile
6) voir exemple 6

On a chauffé à 90° la partie I jusqu'à dissolution complète de tous les ingrédients, puis on a arrêté le chauffage et II a été ajouté au mélange obtenu. Les parties III et IV ont ensuite été ajoutées successivement sous agitation. Le mélange a été enfin versé dans des moules appropriés à 65° environ.

Exemple 9

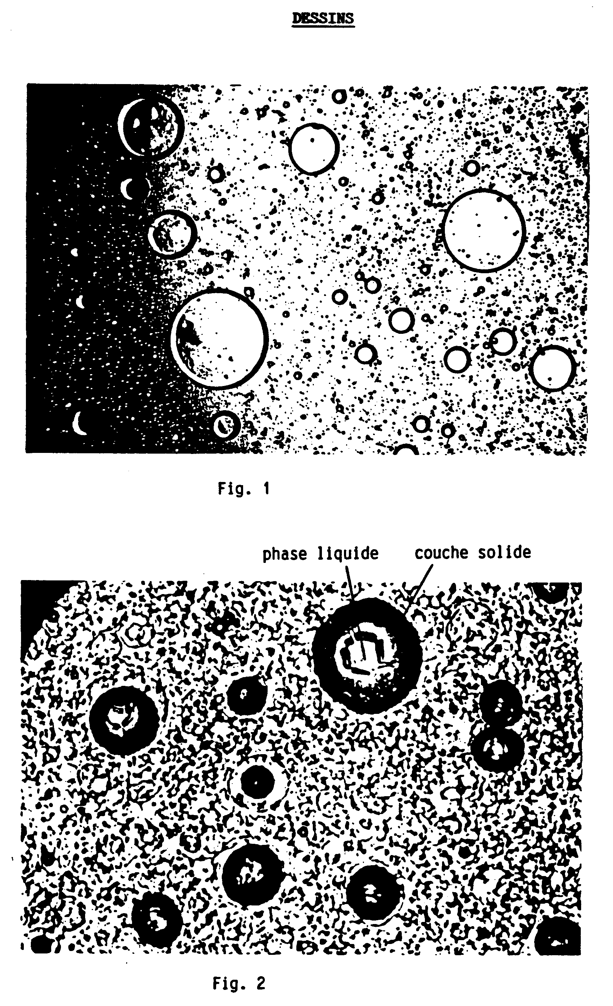

Afin de démontrer l'effet réversible du phénomène de solubilité et "réencapsulation" de la composition selon l'invention, la composition antiperspirante obtenue selon l'exemple 3 a été étalée en couche mince immédiatement après mélange sur un porte-objet et observée au microscope (grossissement 600x). La reproduction de la fig. 1 montre la formation de gouttelettes distinctes d'un diamètre variable. Après 10-20 minutes à l'air, l'émulsion a séché en donnant lieu à la formation d'une membrane solide qui entoure une phase liquide (émulsion) constituée par le parfum (fig. 2).

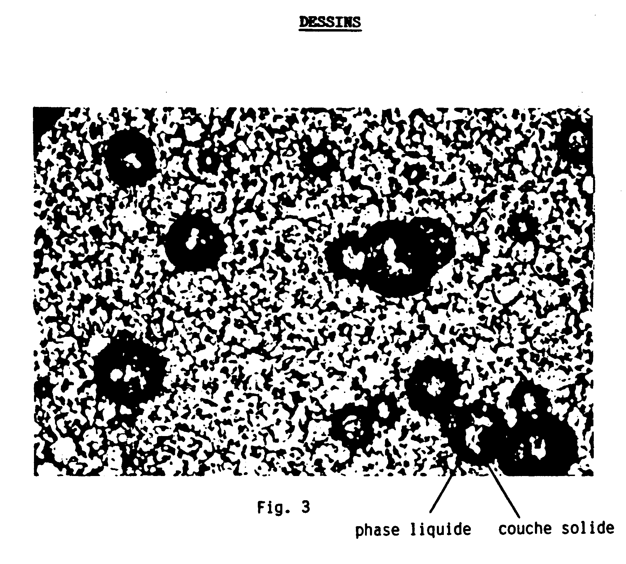

En humidifiant les capsules ainsi formées par l'adjonction de quelques gouttes d'eau, les membranes solides se désagrègent en libérant ainsi la base parfumante liquide qui, au contact direct de l'air, se volatilise en partie dans l'atmosphère environnante. Par un nouveau séchage à l'air, les microcapsules se reforment de sorte que la base parfumante restante est à nouveau entourée, sous forme de gouttelettes, par une membrane solide protectrice (fig. 3).

Un tel phénomène peut être répété plusieurs fois au cours d'une même journée sans qu'on ait observe une altération notable des propriétés du système jusqu'à évaporation complète du parfum.

Un test de stockage prolongé a montré qu'un tel système conserve ses caractères pendant 1 mois au moins.

Le test effectué et décrit cidessus tend à montrer l'une des caractéristiques utiles et inattendues de la composition selon l'invention. Utilisée en effet par aspersion directe sur l'épiderme, la composition sèche d'abord pour donner lieu à la formation de microcapsules renfermant la base parfumante. Par l'action de la sueur, ou au contact d'une source humide, la base parfumante est libérée pour être de nouveau encapsulée in situ une fois terminée la manifestation de transpiration. Le dégagement des constituants volatiles de la base, et par voie de conséquence, l'effet masquant et déodorant de celle-ci, est réellement effectif au moment requis ; l'utilisateur lui-même contrôle en quelque sorte physiologiquement un tel dégagement.

Exemple 10

Une composition antiperspirante, préparée conformément à l'exemple 6, a été appliquée à l'aide d'un spray aérosol dans la région axillaire de 10 sujets mâles d'âge compris entre 21 et 36 ans. L'odeur initiale dégagée par l'épiderme ainsi traité était faible, voire dans certains cas nulle. Après deuxheures environ, les sujets ont été soumis à une activité physique intense, telle celle représentée par 20 minutes de jeu de basket. Une évaluation olfactive réalisée à ce moment-là a permis d'établir que le dégagement de parfum était intense. Pendant la période de pose (5 minutes) qui a suivi, le séchage à l'air de la sueur a entraîné une diminution importante de la diffusion du parfum. A la reprise du jeu, le phénomène de transpiration a provoqué à nouveau le dégagement de parfum qui a été nettement perçu par un panel d'évaluation au terme du jeu pour diminuer ou disparaître après quelques minutes de séchage à l'air.

**Revendications**

**1.** Composition parfumante à action désodorisante ou antiperspirante pour utilisation au moyen d'un dispositif d'application sur la peau, ladite composition contenant une base désodorisante ou antiperspirante active et une base parfumante, cette dernière étant combinée avec un support solide filmogène et un agent émulsifiant pour former une émulsion aqueuse laquelle, lorsque séchée sur la peau, engendre l'encapsulation in situ de la base parfumante, de façon à empêcher la diffusion de celle-ci jusqu'à ce qu'un premier accès de transpiration se produise, suivie de la réencapsulation in situ de ladite base parfumante lors du séchage subséquent de la peau.

**2.** Composition parfumante selon la revendication 1, caractérisée en ce que ledit support solide filmogène est choisi parmi l'acétate de polyvinyle, l'alcool polyvinylique, les dextrines, l'amidon, naturel ou modifié, les gommes végétales, les pectines, les xanthanes, la carboxyméthylcellulose, la méthylcellulose, l'hydroxyéthylcellulose et les lipohétéropolysaccharides.

**3.** Composition parfumante selon la revendication 1 ou 2, caractérisée en ce que ledit agent émulsifiant est choisi parmi les mono- ou diglycérides d'acides gras, les esters dérivés de la combinaison d'acides gras avec le sorbitol ou un mono-saccharide, ou leurs dérivés alcoxylés, ou un ester de l'acide tartrique, citrique, ascorbique ou lactique.

**4.** Composition selon l'une des revendications précédentes, caractérisée en ce que ladite émulsion aqueuse est constituée par
   a. 2 à 20% de support solide filmogène,
   b. 0,1 à 10% d'agent émulsifiant,
   c. 0,1 à 5% de base parfumante,
   d. 5 à 25% de base désodorisante ou antiperspirante active,

le restant étant représenté par de l'eau, des dissolvants inertes et/ou des excipients, le cas échéant, des agents désinfectants, germicides ou bactériostatiques.

5. Composition parfumante selon la revendication 1, caractérisée en ce que la base antiperspirante est constituée par un sel d'aluminium.

6. Composition parfumante selon la revendication 5, caractérisée en ce que le sel d'aluminium est un hydroxychlorure d'aluminium.

7. Dispositif ou article désodorisant ou antiperspirant destiné aux soins corporels caractérisé en ce qu'il contient une composition parfumante selon la revendication 1.

8. Dispositif ou article désodorisant ou antiperspirant selon la revendication 7 choisi parmi les crèmes, les sticks, les roll-ons, les smooth-ons, les aérosols ou les poudres.

**Claims**

1. Perfuming composition with deodorant or antiperspirant action, to be used by means of a device for application on the skin, said composition containing an active deodorant or antiperspirant base and a perfuming base, the latter being combined with a solid film-forming carrier and an emulsifying agent to form an aqueous emulsion, which, once dried on the skin, engenders the in-situ encapsulation of the perfuming base so as to prevent the diffusion of the latter until a first surge of transpiration occurs, followed by the in-situ re-encapsulation of said perfuming base upon the subsequent drying of the skin.

2. Perfuming composition according to claim 1, characterized in that said solid film-forming carrier is chosen amongst polyvinyl acetate, polyvinyl alcohol, dextrins, natural or modified starch, vegetable gums, pectins, xanthanes, carboxymethylcellulose, methylcellulose, hydroxyethylcellulose and lipoheteropolysaccharides.

3. Perfuming composition according to claim 1 or 2, characterized in that said emulsifying agent is chosen amongst the mono- or diglycerides of fatty acids, esters derived from the combination of fatty acids with sorbitol or a mono-saccharide, or their alkoxylated derivatives, or an ester of tartaric, citric, ascorbic or lactic acid.

4. Composition according to one of the preceding claims, characterized in that said aqueous emulsion consists of
   a. 2 to 20% of solid film-forming carrier,
   b. 0.1 to 10% of emulsifying agent,
   c. 0.1 to 5% of perfuming base,
   d. 5 to 25% of active deodorant or antiperspirant base,
   the remainder being water, inert solvents and/or excipients, optionally, desinfecting, germicidal or bacteriostatic agents.

5. Perfuming composition according to claim 1, characterized in that the antiperspirant base is an aluminium salt.

6. Perfuming composition according to claim 5, characterized in that the aluminium salt is an aluminium hydroxychloride.

7. Deodorant or antiperspirant device or article, intended for body-care, characterized in that it contains a perfuming composition according to claim 1.

8. Deodorant or antiperspirant device or article according to claim 7, chosen amongst creams, sticks, roll-ons, smooth-ons, aerosols or powders.

**Patentansprüche**

1. Mittels einer Vorrichtung auf die Haut aufzutragende Riechstoffkomposition mit desodorisierender oder antitranspiratorischer Wirkung, welche eine aktive desodorisierende oder antitranspiratorische Base und eine parfümierende Base enthält; die letztere ist aus einem festen filmbildenden Träger und einem Emulgator zusammengesetzt, um eine wässerige Emulsion zu bilden, welche, wenn auf der Haut getrocknet, eine in-situ Einkapselung der parfümierende Base bewirkt, und somit die Diffusion der Letzteren bis zum ersten Schweißausbruch verhindert, gefolgt von einer erneuten in-situ Einkapselung der parfümierende Base beim nächsten Trocknen der Haut.

2. Riechstoffkomposition gemäß Anspruch 1, dadurch gekennzeichnet, daß der obengenannte feste filmbildenden Träger unter Polyvinylacetat, Polyvinylalkohol, Dextrinen, natürlicher oder modifizierter Stärke, Pflanzenhydrokolloïden, Pektinen, Xanthanen, Carboxymethylzellulose, Methylzellulose, Hydroxyethylzellulose oder Lipoheterosacchariden ausgewählt wird.

3. Riechstoffkomposition gemäß Anspruch 1 oder 2, dadurch gekennzeichnet, daß der obengenannte Emulgator unter Fettsäure-mono- oder -diglyceriden, unter Estern, die aus der Verbindung von Fettsäure mit Sorbitol oder einem Monosaccharid gebildet werden, oder ihren Alkoxyderivaten, oder unter einem Weinsäure-, Zitronensäure-, Ascorbinsäure- oder Milchsäureester ausgewählt wird.

4. Komposition gemäß einem der vorhergehenden Ansprüche, dadurch gekennzeichnet, daß die obengenannte wässerige Emulsion aus
   a. 2 bis 20% des festen filmbildenden Trägers,
   b. 0,1 bis 10% des Emulgators,
   c. 0,1 bis 5% der parfümierenden Base,
   d. 5 bis 25% der aktiven desodorisierenden oder antitranspirantorischer Base,
   und der Rest aus Wasser, inerten Lösungsmitteln und/oder Bindesmitteln, gegebenenfalls Desinfektionsmitteln, keimtötenden oder bakteriostatischen Mitteln besteht.

5. Riechstoffkomposition gemäß Anspruch 1, dadurch gekennzeichnet, daß die desodorisierende oder antitranspiratorische Base aus einem Aluminiumsalz besteht.

6. Riechstoffkomposition gemäß Anspruch 5, dadurch gekennzeichnet, daß das Aluminiumsalz ein Aluminiumhydroxychlorid ist.

7. Desodorisierende oder antitranspiratorische Vorrichtung, oder desodorisierender oder antitranspiratorischer Artikel für Körperpflege, dadurch gekennzeichnet, daß sie oder er eine parfümierende Komposition gemäß Anspruch 1 enthält.

8. Desodorisierende oder antitranspiratorische Vorrichtung, oder desodorisierender oder antitranspiratorischer Artikel, gemäß Anspruch 8, welche(r), unter Cremen, Sticks, Roll-ons, Smooth-ons, Aerosolen oder Pulvern ausgewählt wird.

EP 0 279 328 B1

## DESSINS

Fig. 1

phase liquide    couche solide

Fig. 2

14

DESSINS

Fig. 3

phase liquide    couche solide